(19) Europäisches Patentamt
European Patent Office
Office européen des brevets

(11) EP 4 178 570 B1

(12) EUROPEAN PATENT SPECIFICATION

(45) Date of publication and mention of the grant of the patent:
**28.08.2024 Bulletin 2024/35**

(21) Application number: **21740528.1**

(22) Date of filing: **09.07.2021**

(51) International Patent Classification (IPC):
***A61K 31/357*** *(2006.01)* ***A61P 33/02*** *(2006.01)*

(52) Cooperative Patent Classification (CPC):
(C-Sets available)
**A61K 31/357; A61K 45/06** (Cont.)

(86) International application number:
**PCT/EP2021/069120**

(87) International publication number:
**WO 2022/008708 (13.01.2022 Gazette 2022/02)**

# (54) VETERINARY COMPOSITIONS FOR PREVENTING AND/OR TREATING LEISHMANIOSIS

TIERÄRZTLICHE ZUSAMMENSETZUNGEN ZUR VERHÜTUNG UND/ODER BEHANDLUNG VON LEISHMANIOSE

COMPOSITIONS VÉTÉRINAIRES POUR PRÉVENIR ET/OU TRAITER LES LEISHMANIOSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.07.2020 EP 20305791**

(43) Date of publication of application:
**17.05.2023 Bulletin 2023/20**

(73) Proprietor: **Ceva Santé Animale**
**33500 Libourne (FR)**

(72) Inventors:
- **VARLOUD, Marie**
  **33500 LIBOURNE (FR)**
- **MEDIANNIKOV, Oleg**
  **13005 MARSEILLE (FR)**
- **MEDKOUR, Hacène**
  **34000 MONTPELLIER (FR)**
- **DAVOUST, Bernard**
  **13008 MARSEILLE (FR)**

(74) Representative: **Cabinet Becker et Associés**
**25, rue Louis le Grand**
**75002 Paris (FR)**

(56) References cited:
- **OLIVA GAETANO ET AL: "Guidelines for treatment of leishmaniasis in dogs", JOURNAL OF THE AMERICAN VETERINARY MEDICAL ASSOCIATION, vol. 236, no. 11, 1 June 2010 (2010-06-01), pages 1192 - 1198, XP002801535, ISSN: 0003-1488**
- **CORTES SOFIA ET AL: "In Vitro Susceptibility of Leishmania infantum to Artemisinin Derivatives and Selected Trioxolanes", ANTIMICROBIAL AGENTS AND CHEMOTHERAPY, vol. 59, no. 8, August 2015 (2015-08-01), pages 5032 - 5035, XP002801536**
- **MUTISO JOSHUA MULI ET AL: "In vitro and in vivo antileishmanial efficacy of a combination therapy of diminazene and artesunate against Leishmania donovani in BALB/c mice.", REVISTA DO INSTITUTO DE MEDICINA TROPICAL DE SAO PAULO, vol. 53, no. 3, May 2011 (2011-05-01), pages 129 - 132, XP002801537, ISSN: 1678-9946**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/357, A61K 2300/00**

## Description

### FIELD OF THE INVENTION

The present invention relates to the veterinary field and, more particularly, to the parasitology field in the treatment of leishmaniosis in dogs.

### BACKGROUND OF THE INVENTION

Leishmaniosis (or Leishmaniasis), and more particularly, visceral leishmaniosis (VL) is an infectious, severe and chronic systemic disease. It is a major zoonosis with significant clinical and epidemiological control priority in the world: the domestic dog is the main animal reservoir, while other wild animals as foxes play a role in sylvatic transmission. *Leishmania infantum* (syn. *L. chagasi*) is the causative agent of Canine visceral leishmaniosis (CanL) and human visceral leishmaniosis. In the last few decades, epidemiological changes in VL, including increases in incidence and lethality rate and its spread to new and even urban areas, have been observed. The number of leishmaniosis cases are increasing worldwide. Some reasons are the lack of vaccines, failures in controlling vectors and the increasing selection of parasites resistance to chemotherapy.

CanL is a severe disease that affects several millions of domestic dogs in countries at both shores of Atlantic Ocean (mainly Europe and South America, but spreading in Africa and Asia as well) and may kill infected dogs when left untreated. In the Mediterranean countries, where infection rates are up to 63% including at least 2.5 million seropositive dogs, CanL represents one of the leading causes of death in dogs. It is an expanding disease and it has become one of the most important canine diseases imported to Central Europe.

The range of clinical presentations and immune responses developed by *L. infantum*infected dogs is very wide and variable. Consequently, several forms that can be typified by their numerous clinical signs, including cutaneous, mucocutaneous and visceral forms.

In addition, *L. infantum* infection in dogs can manifest as subclinical infection, as a selflimiting illness or a serious life-threatening disease. Infected dogs may stay for a long time a reservoir and a source for human infections.

Treatment of CanL is a challenge because of the intracellular localization of the parasite. In addition, the World Health Organization (WHO) has suggested reserving antileishmanial drugs used for human VL for exclusive use in human leishmaniosis and not for veterinary medicine, due to suspicion of drug-resistance development from use in animals. Despite this, the first-line treatment for CanL is currently the combined use of leishmanicidal agents used as second-line drugs for humans (e.g., pentavalent antimonials, miltefosine) and allopurinol. Some of the chemotherapeutic compounds used for CanL are included within the 19th edition of WHO Model List of Essential Medicines (April 2015) against leishmaniosis: pentavalent antimonials, miltefosine, amphotericin B deoxycholate or formulated in liposomal formulations, and paromomycin. However, some other drugs are also effective, such as allopurinol, pentamidine, enrofloxacin, marbofloxacin, metronidazole, spiramycin, and ketoconazole. Determination of the best drug is based on clinical examination, and staging of CanL according to immunodiagnostic test results, clinical signs, and clinicopathological abnormalities and parasite load. Most of these treatments are expensive and do not achieve complete cure of the disease; some of them can cause important side effects. Effects as vomiting, lethargy, diarrhea and nephrotoxicity are common during the current treatments with pentavalent antimonials and miltefosine. Prolonged administration of allopurinol induces frequently xanthine urolithiasis. The use of antibiotics against leishmaniosis is also questionable because of the risk of development of resistance in the pathogenic bacteria population. Also, non-treatment of infected dogs puts in jeopardy all humans living in proximity because of the easy transmission of *L. infantum* from reservoir dogs to humans.

Today, the reference protocol for treating CanL uses meglumine antimonate (Glucantime®) and allopurinol (Zyloric®) (Bourdoiseau et Denerolle, Revue Méd. Vét., 2000, 151, 5, 395-400; Solano-Gallego et al., Parasites & Vectors, 2011, 4:86). However, in addition to the side effects of meglumine antimonate and allopurinol, the convenience of the reference protocol is not satisfied since it requires administering of two products and repeated injections for several weeks or even several months. Such treatment protocols represent a risk of compliance failure.

The antiparasitic activity of Artemisinin and derivatives (ARTs) has been slightly explored. For instance, Loo et al., (Pharmacol Res., 2017, 117, 192-217) have reported an efficacy of ARTs in treating protozoan infections beyond malaria (Loo et al., Pharmacol Res., 2017, 117, 192-217). Chollet et al. (Biomedicine & Pharmacotherapy, 2008, 62, 462-465) have reported an *in vitro* activity of fluoroartemisinins against promastigote forms of *L. donovani*. Cortes et al. (Antimicrobial agents and chemotherapy, 2015, 59(8), 5032-5035) have shown that the following compounds, namely, artesunate, deoxygenated artesunate, dihydroartemisinin, deoxygenated dihydroartemisinin exhibited *in vitro* activity against *L. infantum* life stage forms (promastigote and amastigote). However, treatments specific to canine leishmaniosis remain unexplored to date.

There is thus an urgent need for new, efficient, safe, and affordable drugs for the treatment of canine leishmaniosis.

## SUMMARY OF THE INVENTION

In this context, the inventors have surprisingly demonstrated a therapeutic interest of artesunate in the treatment of canine leishmaniosis. More particularly, the inventors have provided a more efficient and tolerant treatment comprising artesunate compared to the reference injectable treatment comprising the combination of meglumine antimonate and allopurinol.

Accordingly, the present invention relates to a veterinary composition comprising artesunate or a salt thereof and a pharmaceutically acceptable excipient, for use for preventing and/or treating leishmaniosis in a dog. More particularly, the dog is a *Leishmania infantum* naturally infected dog.

In a particular embodiment, the veterinary composition for use according to the invention comprises a therapeutically efficient dose comprised between 0.1 and 100 mg/kg BW/day (BW: bodyweight), preferably between 1 and 50 mg/kg BW/day, more preferably between 20 and 30 mg/kg BW/day. In a preferred embodiment, the veterinary composition comprises a therapeutically efficient dose of 25 mg/kg BW/day.

In a further particular embodiment, the veterinary composition for use according to the invention is administered by an oral route. Preferably, the veterinary composition is a tablet.

In a further particular embodiment, the veterinary composition for use according to the invention is administered in a single daily dose. In a preferred embodiment, the veterinary composition is administered for at least 6 days, preferably from 6 to 10 days, more preferably for 6 days.

Another object of the invention is a veterinary composition for use as defined herein for controlling the clinical signs of leishmaniosis and/or *Leishmania infantum* blood load and/or *Leishmania infantum* antibodies.

A preferred object of the invention is a veterinary composition comprising artesunate or a salt thereof and a pharmaceutically acceptable excipient, for use for preventing and/or treating leishmaniosis in a *Leishmania infantum* naturally infected dog, wherein the veterinary composition is administered to the infected dog by oral route in a single daily dose of 25 mg/kg BW/day for 6 days.

In a further embodiment, the veterinary composition for use according to the invention further comprises at least one antiparasitic agent.

Another object of the invention is a kit comprising a first compartment comprising artesunate or a salt thereof, and a second compartment comprising at least one antiparasitic agent as a combined preparation for simultaneous, separate, or sequential use, in particular for preventing and/or treating leishmaniosis in a dog. In a particular embodiment, the kit comprises a notice or an instruction guide.

## LEGEND TO THE FIGURES

**Figure 1:** Consort flow diagram showing the therapeutic protocols and dogs with leishmaniosis in each stage of the study.

**Figure 2:** Time course of total clinical score (Kruskal-Wallis Test). Clinical scores (mean ± SD) of the dogs (n=42) were assessed by veterinarians at the time of scheduled visits at D0, D+30, D+90 and D+180. **A.** All Groups. **B.** Glucantime®/Allopurinol Group. **C.** Artesunate treated Group.

**Figure 3:** Comparison of the mean percentage of reduction in clinical score between Glucantime®/Allopurinol and Artesunate treated groups (Mann-Whitney Test): **A.** Day 30 post treatment; **B.** Day 90 post treatment; **C.** Day 180 post treatment.

**Figure 4:** Monitoring serologic. Antibody titers (mean ± SD) of the dogs (n=42) were assessed by quantitative IF AT at the time of scheduled visits at D0, D+30, D+90 and D+180 (Kruskal-Wallis Test / Bilateral). **A.** All dogs. **B.** Glucantime®/Allopurinol Group. C. Artesunate Group.

**Figure 5:** Comparison of mean percentage of reduction IFAT-antibody titers in dogs with leishmaniosis treated with Artesunate or Glucantime/ allopurinol. Data reported as mean ± SD, Artesunate versus Glucantime/allopurinol (Mann-Whitney test) 30, 90, and 180 days post-treatments.

## DETAILED DESCRIPTION OF THE INVENTION

The references to methods of treatment in this description are to be interpreted as references to the compounds, pharmaceutical compositions and medicaments of the present invention for use in a method for treatment of the human or animal body by therapy.

As illustrated by the examples, the inventors have demonstrated that artesunate was safer and more effective in controlling and reducing the clinicals signs of leishmaniosis, parasite load, and antibodies than meglumine antimoniate/allopurinol in dogs.

The present invention thus relates to artesunate or a salt thereof or a veterinary composition comprising artesunate or a salt thereof, and a pharmaceutically acceptable excipient, for use for preventing and/or treating leishmaniosis in a dog. The invention further relates to a veterinary composition comprising artesunate or a salt thereof, and a pharmaceutically acceptable excipient, for use for preventing and/or treating canine Leishmaniosis. (CanL). The invention also relates to a veterinary composition comprising artesunate or a salt thereof, and a pharmaceutically acceptable excipient, for use for preventing and/or treating canine Leishmaniosis in a *Leishmania infantum* naturally infected dog. In a particular embodiment, leishmaniosis is visceral leishmaniosis. In a further particular embodiment, canine Leishmaniosis (CanL) is visceral canine Leishmaniosis.

The invention further concerns a method for treating leishmaniosis in a dog, comprising administering an effective amount of artesunate or a salt thereof, or a veterinary composition comprising the same, in a dog, preferably in a *Leishmania infantum* naturally infected dog. The invention also concerns a use of artesunate or a salt thereof, for the manufacture of a pharmaceutical composition for preventing and/or treating leishmaniosis in a dog, preferably in a *Leishmania infantum* naturally infected dog.

Artesunate is a sesquiterpene-type organic peroxide, and more particularly, a semisynthetic derivative of the artemisinin group having the following formula:

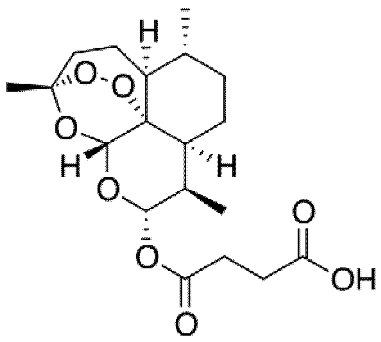

.

Artesunate is currently administered intramuscularly, intravenously, or orally (per os) as a first-line treatment for severe malaria. For instance, it is used in combination products commercialized by Denk pharma with other antimalarial agents, namely pyrimethamine and sulfamethoxypyrazine, or amodiaquine.

Within the context of the invention, the salts of artesunate correspond to its pharmaceutically acceptable salts. A pharmaceutically acceptable salt includes inorganic as well as organic acids salts. Representative examples of suitable inorganic acids include hydrochloric, hydrobromic, hydroiodic, phosphoric and the like. Representative examples of suitable organic acids include formic, acetic, trichloroacetic, propionic, benzoic, cinnamic, fumaric, maleic, methanesulfonic and the like.

As used herein, the expression "effective dose" or "effective amount" or "therapeutically efficient dose" means the dose or the quantity sufficient for preventing and/or treating a leishmaniosis. A skilled person is able to adapt the "effective dose" or the "effective amount" or the "therapeutically efficient dose" according to the severity of leishmaniosis and the general health of the dog.

In a particular embodiment, the veterinary composition for use according to the invention comprises a therapeutically efficient dose comprised between 0.1 and 100 mg/kg BW/day, preferably between 1 and 50 mg/kg BW/day, 10 and 40 mg/kg BW/day, more preferably between 20 and 30 mg/kg BW/day. "BW" means "bodyweight". In a preferred embodiment, the therapeutically efficient dose is comprised between 21 and 29 mg/kg BW/day, 22 and 28 mg/kg BW/day, 23 and 27 mg/kg BW/day, and 24 and 26 mg/kg BW/day. In a more preferred embodiment, the therapeutically efficient dose is 25 mg/kg BW/day.

The veterinary composition may be administered one or several takes a day, preferably, one or two takes a day, more preferably one take a day. A preferred object of the invention is thus a veterinary composition for use, wherein the veterinary composition is administered in a single daily dose or in a single daily take.

The treatment as defined herein may be applied each day for a limited period, for instance one month (or 30 days), and preferably for a short time of 25, 20, 15, 10, 9, 8, 7, 6, 5, 4, 3, 2, or 1 day. In a particular embodiment, the veterinary composition for use according to the invention is administered for at least 6 days, preferably from 6 to 10 days, from 6 to 9 days, from 6 to 8, more preferably for 6 days. Preferably, a period defined by a number of days from X to Y, means that the treatment is applied in a minimum of X days and a maximum of Y days. In other words, the treatment is applied for at least X days and at most Y days.

As used herein, a "pharmaceutically acceptable excipient" or a "veterinary acceptable excipient" is an ingredient or a compound conventionally used in the pharmaceutical or veterinary field for the preparation of pharmaceutical or

veterinary compositions and formulations according to the administration route. Preferably, such an acceptable excipient does not have a therapeutic effect. Without limitation, a pharmaceutically or veterinary acceptable excipient includes buffer agents, preservatives, antioxidants, diluents, stabilizers, tonicity agents, solubilization agents, suspension agents, surfactants, lubricants, and solvents.

Without limitation, examples of solvents are propylene glycol, ethanol, sorbitol syrup, water, vegetable oils, or combinations thereof, and the like. Examples of buffer agents are calcium phosphate, sodium bicarbonate, sodium phosphate, sodium lactate, sodium citrate, or combinations thereof, and the like. Examples of suspension agents include methylcellulose, hydroxypropylmethylcellulose, polysorbate 80, hydroxyethylcellulose, hydroxycellulose, anthan gum, sodic carboxymethylcellulose and polyethoxylated sorbitan monolaurate, or combinations thereof, and the like. Examples of stabilizers include sodium sulfite, sodium metalsulfite and ether. Examples of tonicity agent are mannitol, sodium chloride, sodium citrate glycerin, lactose, mannitol, sodium chloride, dextrose, sodium sulfate, sorbitol, or combinations thereof, and the like. Examples of preservatives are ethyl p-hydroxybenzoate, w-propyl p-hydroxybenzoate, methyl p-hydroxybenzoate, sorbic acid, phenol, cresol, chlorocresol, benzoic acid or salts thereof, sodium metabisulfite, sodium benzoate, benzethonium chloride, cetylpyridinium chloride, benzalkonium chloride, sodium acetate, parabenes and salts thereof, sucrose, or combinations thereof, or combinations thereof, and the like. Examples of solubilization agents include polyoxyethylene-solidified castor oil, polysorbate 80, nicotinamide, polyethoxylated sorbitan monolaurate, macrogol and ethyl ester of caste oil fatty acid, or combinations thereof, or combinations thereof, and the like. Examples of diluents are calcium carbonate, calcium phosphate, kaolin, sodium carbonate, lactose, sodium phosphate, lactose monohydrate, or combinations thereof, and the like. Examples of antioxidants include: ascorbic acid (vitamin C) or salts thereof, citric acid or salts thereof, malic acid, monothioglycerol, fumaric acid, phosphoric acid, sodium metabisulfite, potassium metabisulfite, propionic acid, sodium bisulfite, sodium metabisulfite, potassium metabisulfite, vitamin E (tocopherol), acetone, cysteine, gentisic acid ethanolamine, glutamate monosodium, formaldehyde sulfoxylate sodium, or combinations thereof, and the like. Examples of lubricant include oils, waxes, stearic acid, stearates (magnesium stearate, calcium stearate...), talc, long chain fatty acids, polyethylene glycol, palmitic acid, hydrogenated vegetable oils, sodium stearyl fumarate, sodium benzoate, and mixtures thereof. Examples of surfactants include poloxamer, polysorbate, polyoxyethylene hydrogenated castor oil...) and mixtures thereof.

The veterinary composition for use according to the invention may be administrated to the dog by any routes, such as oral route, parenteral route including intramuscular and subcutaneous injection, and topical route. In a preferred embodiment, the veterinary composition is administered by an oral route. Such an oral administration is more convenient for the user (proprietor of the dog or the veterinary) compared to the injectable administration meglumine antimoniate used in the reference treatment with allopurinol.

The veterinary composition for use according to the invention may be formulated in any forms, such as a form of a liquid solution, suspension, solid or semi-solid, powder, pellet, capsule, granule, sugar-coated pill, capsule, spray, pill, tablet, paste, implant or gel. For an oral administration, a liquid solution, a suspension, a solid or semi-solid, powder, a chew, a pellet, a capsule, a granule, a sugar-coated pill, or a tablet are preferred. In a more preferred embodiment, the veterinary composition for use according to the invention is a tablet. A tablet suitable for implementing the invention is the artesunate tablet commercialized under the trademark AsunateDenk®.

For a formulation in a tablet or a pellet suitable for an oral administration, monohydrate lactose, magnesium stearate, microcrystalline cellulose, polyvinylpyrrolidone, crospovidone, aromas and compressible sugars can be used as excipients, in a solvent.

A preferred object of the invention is a veterinary composition comprising artesunate or a salt thereof, and a pharmaceutically acceptable excipient, for use for preventing and/or treating leishmaniosis in a *Leishmania infantum* naturally infected dog, wherein said veterinary composition is administered to said infected dog by oral route in a single daily dose of 25 mg/kg BW/day for 6 days. A further preferred object of the invention is a method for treating leishmaniosis in a *Leishmania infantum* naturally infected dog comprising orally administering to said dog a veterinary composition comprising artesunate or a salt thereof, and a pharmaceutically acceptable excipient, in a single daily dose of 25 mg/kg BW/day for 6 days.

As used herein, the term "treatment" or "treating" refers to any act intended to ameliorate the health status of said dog such as therapy, prevention, prophylaxis, retardation, and limiting the development of leishmaniosis as disclosed herein. In certain embodiments, such term refers to the amelioration or eradication of leishmaniosis due to a parasitic infestation, such as *Leishmania infantum,* or symptoms associated with such disease. In other embodiments, this term refers to minimizing the spread or worsening of Leishmaniosis due to the parasitic infestation resulting from the administration of compositions as disclosed herein to an infested dog.

The term "treatment" or "treating" also includes the preventive treatment of a dog against leishmaniosis due to a parasite infestation, which designates a treatment performed before the dog has been exposed to or in contact with the causative agent of the disease such as a parasite, or after said exposure/contact but before development of the symptoms or at an early stage of development of the disease due to a parasite infestation. In a particular embodiment, the term "treatment" or "treating" designates the protection of a dog against leishmaniosis, e.g., against the effect of an

exposure to the causative agent, or against the development of leishmaniosis in exposed dogs. The invention particularly suits to protect dogs against leishmaniosis due to a parasitic infection or infestation. An object of the invention is therefore a method for protecting a dog against leishmaniosis, comprising administering an effective amount or an effective dose of a veterinary composition as disclosed herein in a dog.

The term "treatment" or "treating" also includes the alleviation of the symptoms such as a decrease of clinical symptoms or clinical manifestations such as general conditions, in particular emaciation, anorexia, apathy, anaemia, and hyperthermia, cutaneous signs, in particular localized alopecia, pruritus, squamose, hyperkeratosis, onychogriphosis, and ulcers, visceral lesions, in particular lymphadenopathy, splenomegaly, hepathomegaly, and renal disorders, ophthalmic conditions, in particular depilation around the eyes "in glasses", mucopurulent conjunctivitis, keratitis and/or uveitis, oral conditions, in particular oral erosions, skeletal conditions, in particular arthritis, synovitis, and diffuse pain of the posterior train, and other conditions such as epistaxis, chronic diarrhea, and neurological signs. Other symptoms are parasite blood load and antibodies, more particularly *Leishmania infantum* blood load *Leishmania infantum* and antibodies.

Another object of the invention is a veterinary composition for use as defined herein for controlling the clinical signs of leishmaniosis. A further object, is a method for controlling the clinical signs of leishmaniosis in a dog comprising administering a veterinary composition as defined herein.

Another object of the invention is a veterinary composition for use as defined herein for controlling *Leishmania infantum* blood load. A further object, is a method for controlling *Leishmania infantum* blood load in a dog comprising administering a veterinary composition as defined herein.

Another object of the invention is a veterinary composition for use as defined herein for controlling *Leishmania infantum* antibodies. A further object, is a method for controlling *Leishmania infantum* antibodies in a dog comprising administering a veterinary composition as defined herein.

As used herein, the term "control" and "controlling" mean without limitation, reducing, limiting the development, eradicating, decreasing, and eliminating. In a particular embodiment, such terms mean a prophylactic treatment of infected dogs, preventing thereby human infections by *L. infantum.*

As used herein, the term "comprise(s)" or "comprising" (and other comparable terms, e.g., "containing," and "including") is "open-ended" and can be generally interpreted such that all of the specifically mentioned features and any optional, additional and unspecified features are included. According to specific embodiments, it can also be interpreted as the phrase "consisting essentially of" where the specified features and any optional, additional and unspecified features that do not materially affect the basic and novel characteristic(s) of the claimed invention are included or the phrase "consisting of" where only the specified features are included, unless otherwise stated.

Thus, the veterinary composition may further comprise at least one antiparasitic agent. These antiparasitic agents comprise in particular insect growth regulators, antibiotic pesticides, botanical pesticides, organophosphorus compounds, carbamates, amidines, organochlorinated compounds, phenylpyrazole compounds, pyrethroids, benzylureas, pyrethrinoids, formamidines, semicarbazones, neonicotinoids, diamides, spinosyns, isoxazolines, copper-containing pesticides, anthelmintic agents, benzimidazoles, probenzimidazoles, salicylanilides, imidazothiazoles, amino-acetonitrile derivatives (AADs), spiroindoles, pyrimidines, substituted phenols, and anticoccidials.

For example, the insect growth regulator may be a chitin synthesis inhibitor or a synoptic juvenile growth hormone, such as azadirachtin, bistrifluron, diofenolan, fenoxycarb, hydroprene, kinoprene, methoprene, pyriproxyfen, tetrahydroazadirachtin, chlorfluazuron, cyromazine, diflubenzuron, fluazuron, flucycloxuron, flufenoxuron, hexaflumuron, lufenuron, novaluron, noviflumuron, tebufenozide, teflubenzuron, or triflumuron. The antibiotic pesticide may be a *Bacillus thuringiensis subsp. israelensis* toxin. The botanical pesticides may be d-limonene, thymol, geraniol, linalol, carvacrol, nicotine, ryania or pyrethrins. The organophosphorus compounds may be chosen from dicrotophos, terbufos, dimethoate, diazinon, disulfoton, trichlorfon, azinphos-methyl, chlorpyrifos, malathion, oxydemeton-methyl, methamidophos, acephate, ethyl-parathion, parathion-methyl, mevinphos, phorate, carbofenthion, phosalone, naphthalophos or pyraclofos. The carbamates may be chosen from carbaryl, carbofuran, aldicarb or carbofuran. The organochlorinated compounds may be chosen from methoxychlor, dicofol or a cyclodiene such as endosulfan. The pyrethrinoids may be chosen from allethrin, resmethrin, permethrin, flumethrin, deltamethrin and cypermethrin. An example of formamides is amitraz. An example of semicarbazones is metaflumizone. The neonicotinoids may be chosen from imidacloprid, nitenpyram or dinotefuran. The copper-containing pesticide may be copper(II), or a copper oxychloride hydroxide or sulfate, namely ($Cu_2Cl(OH)_3$) mixed with ($Cu_4(OH)_6(SO_4)$). The anthelmintic agent may be chosen from a macrocyclic lactone such as an avermectin (for example ivermectin and moxidectin) or a milbemycin (for example milbemycin oxime). The benzimidazoles may be albendazole or thiabendazole. The salicylanilides may be closantel or oxyclozanide. Levamisole may be one of the imidazothiazoles. Pyrantel may be one of the pyrimidines. Nitroxynil may be one of the substituted phenols. Fipronil may be one of the phenylpyrazoles. The isoxazolines may be 4-{ 5-[3-chloro-5-(trifluoromethyl)phenyl]-5-(trifluoromethyl)-4,5-dihydroisoxazol-3-yl}-N-{2-oxo-2-[(2,2,2-trifluoroethyl)amino]ethyl}naphthalene-1-carboxamide (afoxolaner), 4-[5-(3,5-dichlorophenyl)-5-(trifluoromethyl)-4,5-dihydro-1,2-oxazol-3-yl]-2-methyl-N-{2-oxo-2-[(2,2,2-trifluoroethyl)amino]ethyl}benzamide (fluranaler) or N-{2-chloro-{5-(3,5-dichlorophenyl)-5-trifluoromethyl-4,5-dihydroisoxazol-3-yeenzyl } cyclopropanecarboxylic.

The various classes of parasites and classes of antiparasitic agents and also the active agents sold can be found on the parasitipedia website at the following address: http://parasitipedia.net/; and on the European Scientific Counsel Companion Animal Parasite (ESCCA) website at the following address: http://www.esccap.org/.

A further object of the invention is a kit comprising a first compartment comprising artesunate or a salt thereof, and a second compartment comprising at least one antiparasitic agent as defined herein as a combined preparation for simultaneous, separate, or sequential use, in particular for preventing and/or treating leishmaniosis in a dog. In a particular embodiment, the kit comprises a notice or an instruction guide.

As used herein, the terms "separate" or "sequential" mean that artesunate or a salt thereof and the at least one antiparasitic agent are administered successively. For instance, artesunate or a salt thereof is firstly administered in a dog, and the at least one antiparasitic agent is secondly administered in the same dog, or vice versa. In this context, artesunate or a salt thereof and the at least one antiparasitic agent are physically sufficiently distinct from being separately or sequentially administrable.

In another aspect, the veterinary composition for use according to the invention comprises no further antiparasitic agent. According to this embodiment, artesunate is the sole active ingredient against leishmaniosis in the veterinary composition. A further object of the invention is, therefore, a veterinary composition consisting essentially of artesunate or a salt thereof, and a pharmaceutically acceptable excipient, for use for preventing and/or treating leishmaniosis in a dog. A further object of the invention is a veterinary composition consisting of artesunate or a salt thereof as a sole active ingredient, and a pharmaceutically acceptable excipient, for use for preventing and/or treating leishmaniosis in a dog.

Further aspects and advantages of the invention will be disclosed in the following experimental section. The examples below illustrate the present invention and are given as non-limiting illustrations.

## EXAMPLES

### Material and Methods

#### Ethic statement

Protocol of the study was also approved by the scientific college (Procès-Verbal du CSI N°6, 2018) at the Veterinary Science Institute, University Constantine 1, Algeria. To facilitate field work, collaborations were established with veterinary doctors and their assistants working in these establishments. All dog owners gave their verbal informed consent and samples were collected by a veterinarian specialist. Risk assessment was also submitted to and approved by the ethics committee and decision board of the veterinary practitioners from the wilayas of the North of Algeria. These institutions are affiliated with the Algerian Ministry of Agriculture and Rural Development (Directions des Services Vétérinaires).

#### Study

This was an open-label randomized comparative clinical field trial conducted in the north of Algeria. Prevalence observed in this area was 36%. Client-owned dogs of any age, breed, or gender were enrolled and 11 veterinary practitioners were responsible of the monitoring. All dogs had previously been naturally infected by *L. infantum* and presented clinical signs. The dogs were fully monitored and treatment could be changed to antimoniate meglumine/allopurinol at the owner's request if clinical impairment was noticed. A total of 187 dogs were enrolled for CanL. Among them, 42 adult sick dogs (30 males and 12 females), 3.8 years average age (min=1, max=11 years), weighing between 3-37 kg and of different breeds (8 german shepherd, 1 belgian shepherd malinois, 2 sloughy, 5 pointers, 6 braques, 1 bagnole, 1 americain staff, 1 beagle and 17 mixed breeds), were selected for this study.

#### Inclusion, exclusion and efficacy criteria

Main inclusion criteria were: i) Dogs with at least two clinical manifestations related to CanL as detailed in the following Table 1) and ii) Dogs with positive serology for *Leishmania* by the immunochromatographic based qualitative rapid test WITNESS® *Leishmania* (Zoetis, France) and/or the quantitative indirect immunofluorescence antibody test (IFAT≥ 100) or positive PCR result obtained in blood.

Dogs with significant alterations in renal and/or hepatic function or with other infectious diseases were excluded. Dogs were excluded if they had a concomitant disease that might interfere with evaluation of response to treatment, if they had been vaccinated against or had been treated with a leishmanicidal for 3 months or with a leishmanistatic for 1 month before pre-inclusion. Finally, no previous treatment with systemic antibiotics or antifungals within 7 days or with systemic long-acting corticosteroid within 1 month prior to pre inclusion was allowed. Pregnant and lactating bitches

were excluded. Dogs could be withdrawn from the study at any time if they showed intolerance to the treatment or if requested by the owner.

Efficacy criteria were determined by reduction in clinical scores (i.e. the sum of clinical scores was lower than before treatment), in decreasing of parasite DNA and *Leishmania*specific antibody titers. The score was defined according to the severity of each clinical sign and the final value was obtained from the sum of all the values (Table 1). Quantitative parasite load was estimated through qPCR and the antibody titers through quantitative IFAT.

**Table 1:** Clinical signs related to CanL defined to evaluate the clinical scores of dogs.

| Symptoms | Number of signs | Clinical manifestations |
|---|---|---|
| **General conditions** | 5 | Emaciation, anorexia, abbatement, anaemia, hyperthermia |
| **Cutaneous signs** | 6 | Localized alopecia, pruritus, squamose, hyperkeratosis, onychogriffose, ulcers |
| **Visceral lesions** | 4 | Lymphadenopathy, splenomegaly, hepathomegaly, renal disorders |
| **Ophthalmic level** | 4 | Depilation around the eyes "in glasses", mucopurulent conjunctivitis, keratitis and/or uveitis |
| **Oral level** | 1 | Oral erosions |
| **Skeletal level** | 3 | Arthritis, synovitis, diffuse pain of the posterior train |
| **Others** | 3 | Epistaxis, chronic diarrhea, neurological signs |

Each manifestation was assigned a severity score ranging from 0 to 3, where 0 = none, 1 = mild, 2 = moderate, and 3 = severe (maximum total clinical score = 21). Mortality related to CanL was assessed by the highest score (22).

**Experimental design**

Selected dogs were included randomly to one of the two treatment arms. Dogs (N=26) in the **Group 1:** (positive control) received 100 mg/kg Antimoniate of meglumine (Glucantime®, Merial, France) daily for 28 days subcutaneously, and 30 mg/kg allopurinol (Allopurinol Arraw® 300 mg, France) orally daily for one month. Dogs (N=16) in the **Group 2:** (chemotherapy of the invention) received 25 mg/kg artesunate (Asu-Denk®; Denk Pharma, Germany) orally for 6 days. After each administration, the dogs were observed for 30 min to monitor for reactivity, vomiting and/or regurgitation to ensure complete absorption of the drug. Dogs were followed up for 180 days (Figure 1).

***Visit schedule and sample collection***

Animals were checked on days D0, D+30, D+90 and D+180 after the treatment had started and blood samples were collected each time-points. Clinical signs and lesions associated with leishmaniosis, infection status (parasite load-qPCR and serological) were evaluated and monitored in these points.

***Clinical evaluation***

The efficacy of treatments was assessed at each time-point based on the clinical response to treatment (evolution of clinical scores over time and the percentage reduction of the total clinical score). For each dog, the same veterinarian conducted all clinical evaluations to maintain consistency. Clinical scores were classified according to the severity of clinical signs listed in Table 1. Mortality due to CanL constitutes the higher score (22). The percentage of reduction was calculated according to the formula:

$$\text{Percentage reduction of CS \%} = (\text{CS at D+XX} - \text{CS at D+0}) / \text{CS at D+0}) \times 100$$

***Parasite load-qPCR***

Blood samples collected in each time-point were analyzed for quantification of parasitic load by qPCR using primers and probe for detection and quantification of *L. infantum* DNA, targeting a conserved region of the kinetoplast minicircle DNA (kDNA) (several 1000's-fold repeated sequence). Prior to DNA extraction, 200 μL of blood were digested with proteinase K and incubated at +56°C overnight. Extraction was performed using a commercial DNA extraction kit (QIAamp DNA Mini Kit®, [Qiagen, Courtaboeuf, France]) and performed on BIOROBOT EZ1 (Qiagen, Qiagen, Courtaboeuf, France) per the manufacturer's instructions. DNA was eluted in 200 μl of distilled water and stored at -20°C until analysis. qPCR was prepared and performed as follows: 20 μL final volume containing 10 μL of Eurogentec Master Mix Roche (Eurogentec, Liège, Belgium), 0.5 mM of each primer and the probe, 0.5 μL UDG, 3 μL of distilled water DNAse and RNAse free, and 5 μl of the DNA sample, was amplified in a CFX96 Real-Time system (BioRad Laboratories, Foster City, CA, USA) using the following thermal profile: one incubation step at 50°C for two minutes and an initial denaturation step at 95°C for three minutes, followed by 40 cycles of denaturation at 95°C for 15 seconds and annealing extension at 60°C for 30 seconds. Quantification was based on a standard curve which was 8 folds-serial dilution of plasmid of $10^8$ copy of DNA/mL from the kDNA region, equivalent of 10000 parasites/mL; 5 μl of serial dilutions ranging from 10000 to 0.001 parasites/mL, was introduced into reaction tubes. Results were expressed as the number of *Leishmania* parasites present in 1 ml of blood, taking in account the volume (200 μl of blood) and the elution (200 μl) introduced during the extraction process.

***Serological monitoring***

At day 0, the immunochromatographic based qualitative rapid test WITNESS® *Leishmania* (Zoetis, France) was performed according to the manufacturer's instructions using one drop (10 μL) of whole blood from each dog directly after sampling. Quantitative IFAT for the titration of anti-*L. infantum* specific immunoglobulins G (IgG) was performed on sera for the monitoring as described previously. Briefly, plate wells were sensitized by 20 μL of *L. infantum* commercial antigens (Zoetis, France). After dilution to 1:50, 20 μl of every serum dilution was applied per well and plates were incubated for 30 min at 37 °C. Plates were washed twice with PBS for 5 min and once with distilled water. 20 μL of IgG anti-dog conjugated with fluorescein isothiacyanate (FITC) (Sigma-Aldrich, St Louis, MO, USA) were added into each well. The plates were incubated for 30 min at 37 °C in the dark. After another washing step, some drops of mounting medium were added on the cover slips and reading was performed. To avoid errors of observation, all samples were examined by two different investigators and positive and negative controls were added in each plate. All samples negative at 1:50 were considered negative. Positive results were further investigated using a five-fold serial dilution IFAT at 1:100 to 1:1600. At this point, the samples were classified as a high positive for *L. infantum* (> 1:1600).

**Data analysis**

XLSTAT Addinsoft version 2018.7 was used for the statistical analysis. A descriptive analysis of the data was performed according to the nature of the variables for each follow-up visit and assigned treatment. The non-parametric Kruskal-Wallis / bilateral Test was used for comparison of averages taken at evaluation time-points for both groups, Group 1, and Group 2 alone. A Multiple pairwise comparisons following the Dunn / Bilateral Test procedure was used for the CS, parasitemia and antibody titers. Treatment effects were compared between the two groups by analysis of percentage reduction of clinical signs, parasite load and Leishmania-antibodies. Mann-Whitney test was used to compare efficacy of treatments using the difference between D0 and D+30 days, D0 and D+90 days and D0 and D+180 days. The significance level used for the tests was 5% (Corrected level of significance of Bonferroni: 0.0083).

**Results**

**Included dogs and monitoring**

No statistically significant difference had been found on age, gender, breed (mongrel or breeder), clinical status of dogs, means of parasitemia and Leishmania-antibody titers between the two groups at the inclusion day (P-value> 0.05).

Clinical examinations of the selected dogs revealed the presence of the general signs in 92.85% (39/42), cutaneous in 83.33% (35/42), visceral in 38.1% (16/42), ophthalmic in 23.8% (10/42), musculo-skeletal in 4.76% (2/42). One dog (2.30%) showed an oral erosion, one other presented epistaxis and two dogs (4.76%) had neurological disorders.

**Table 2:** Characteristics of dogs assigned to each treatment arm and homogeneity analysis data, expressed as mean ± standard deviation and frequencies (%)

| Variable | Treated Group 1 (n=26) | Treated Group 2 (n=16) |
|---|---|---|
| **Age, year** | 3.71 ± 2.11 | 3.94 ± 1.45 |
| (min- max) | (1- 11) | (2.5- 8) |
| **Gender, male (%)** | 17 (65.39) | 13 (81.25) |
| **Breed** | | |
| Breeder (%) | 17 (65.39) | 8 (50) |

(continued)

| | | |
|---|---|---|
| **Breed** | | |
| Mongrel (%) | 9 (34.61) | 8(50) |
| **Clinical score:** points | 7.46 ± 4.04 | 6.67 ± 2.30 |
| (min- max) | (3- 20) | (3- 11) |
| **No. of positive by PCR (%)** | 7 (26.92) | 5 (31.25) |
| **qPCR-Parasitemia (Leish/mL)** | 1108.72 ± 4154.5 | 78.04 ± 98.98 |
| (min- max) | (0- 21000) | (0- 233) |
| No. Positive PCR (%*) | 7 (26.92) | 5 (25) |
| **Leishmania Atibody titers, IFAT:** | 476.92 ± 448.38 | 390.62 ± (507.35) |
| (min- max) | (100- 1600) | (50- 1600) |

%*: (Number of positive by PCR/Number of total positive) x 100.

Statistical analysis was performed on 42 dogs, as one dog from Group 2 was lost to follow-up before the D+30 time-point. During the 6-month follow-up, four dogs from the Group 1 were lost to follow-up two months after the beginning of treatments, two of them due to failure of clients to return for the final re-visits on D+90 and D+180. Two others because of death not relative to CanL. In addition, six dogs were died in the period between the D+60 after the beginning of treatments and before the visit D+90, with increasing in the clinical signs related to CanL. Four of them were in the Group 1 and two were in the Group 2 (Figure 1).

**Clinical outcome**

An improvement of the total clinical scores in both treatment groups was generally observed throughout the study compared to the initial clinical score. With given to the mortality outcome the higher score (22), mean of clinical scores decreased over time from 7.16± 3.47 at D0 to 4.24± 4.37 at the visit D+ 30, re-increased a little at D+ 90 (5.75± 8.25) but they remain inferior to D0 and steal constant (5.60± 8.35) after that at D+ 180. The same kinetics of clinical scores were observed in the two groups separately (Figure 2).

Taking the clinical score (CS) at D0 and D+30 as the starting and end point, respectively, the mean percentage of reduction of clinical score was 43.3+ 47.8%. At each point-time: D+30, D+90 and D+180, statistically significant difference ($p<0.0001$) for the mean percentage of reduction of clinical score was observed between the two groups. More improvement has been noted in the Group 2 (Artesunate)than the Group 1 (Glucantime®/Allopurinol) (Figure 3). At the level of individual responses, approximately 26.66% (4/15) of Group 2 dogs gained a complete recovery (CS of 0) versus 18.18 (4/22) in the Group 1, while approximately half of the dogs showed at partial amelioration 6 months following up in both groups with 54. 54% (12/22) and 46.66 (7/15) in control and artesunate group respectively. At D+180, clinical aggravation rates were closely similar in both groups with 27.27% (6/22) including 18.18% (4/22) of mortality in the control group versus 26.66% (4/15) of aggravation including 13.33% (2/15) of mortality in the artesunate group. Details on clinical changes over time for the followed dogs are described in the Table 3.

**Adverse events**

No major side effects related to these treatments were reported in any dog. Both study compounds were well tolerated in all dogs except two dogs which had slight local pains and pruritus, 15 days of treatment with Glucantime®/ allopurinol.

**Table 3. Individual clinical changes on dogs over time.**

| GROUPS | VISIT | COMPLETE AMELIORATION (%) | PARTIAL AMELIORATION (%) | NO CHANGE (%) | AGGRAVATION (%) |
|---|---|---|---|---|---|
| **ARTESUNATE GROUP** | D+30 (n=15) | - | 12(80) | - | 3(20) |
| | D+90 (n=15) | 3(20) | 8(53.33) | 1 (6.66) | 3(20) |
| | D+180 (n=15) | 4(26.66) | 7(46.66) | - | 4(26.66) |

(continued)

| GROUPS | VISIT | COMPLETE AMELIORATION (%) | PARTIAL AMELIORATION (%) | NO CHANGE (%) | AGGRAVATION (%) |
|---|---|---|---|---|---|
| GLUCANTIME/ ALLOPURINOL GROUP | D+30 (n=26) | 2(7.69) | 17(65.38) | 1(3.84) | 6(23.07) |
| | D+90 (n=23) | 3(13.(4) | 13(56.52) | 1(4.34) | 6(26.08) |
| | D+180 (n=22) | 4(18.18) | 12(54.54) | - | 6(2727) |
| TOTALDOGS | D+30 (N=41) | 2(4.87) | 29(70.73) | 1(2.44) | 9(21.91) |
| | D+90 (N=38) | 6(15.79) | 21(5526) | 2(526) | 9(23.68) |
| | D+180 (N=37) | 8(21.62) | 19(51.35) | - | 10(27.02) |

Mortality related to CanL was included

**Quantitative real-time PCR kinetic parasitemia**

At D0, 12/42 (28.5%) of dogs were positive by qPCR in blood. A parasitic clearance was observed over-time after treatment with artesunate and all initially positive dogs (5/5) turned out to be *Leishmania-negative.* Whereas, 6/7 PCR-positive dogs treated with Glucantime®/ allopurinol turned out to be negative and 1/7 dog remained positive. In addition, two dogs treated with Glucantime®/ allopurinol was found positive whereas they were negative before. Individual evolution of parasitemia over the follow up is showed in the Table 4.

**Table 4. Parasite load, Number of parasites per mL of blood and percentage of reduction over time.**

| Dog's ID | Treatment | Parasitemia No. Leish/mL of blood (% of reduction) | | | |
|---|---|---|---|---|---|
| | | D0 | D+30 | D+ 90 | D+ 180 |
| **13** | Glucantime/ Allopurinol | 1500 | 288,7 (80,75) | 30,28 (97,98) | 357 (76,2) |
| **5** | | 0 | 0 (0,00) | 176 (-100) * | 177 (-100) * |
| **A11N12** | | 0 | 46,6 (-100)* | N/A* | N/A* |
| **S26** | | 2400 | 19,8 (99,18) | 2 (99,92) | 0 (100) |
| **S27** | | 3800 | 0 (100) | 0 (100) | 0 (100) |
| **S33** | | 21000 | 0 (100) | 0(100) | N/A |
| **S54** | | 7,7 | 0 (100) | 0(100) | N/A |
| **C4P2G2** | | 101 | 16,2 (83,96) | 0 (100) | 0 (100) |
| **C1G2P4** | | 18 | 0 (100) | 0(100) | 0 (100) |
| **10** | Artesunate | 6,8 | 0 (100) | 0(100) | 0 (100) |
| **S50** | | 17,4 | 14 (19,54) | 29 (-66,67) | 0 (100) |
| **A9N9** | | 122 | 44 (63,93) | N/A | N/A |
| **T11 C2P1** | | 233 | 1,7 (99,27) | 0 (100) | 0 (100) |
| **A8 N8** | | 11 | 0,17 (98,45) | 0 (100) | 0 (100) |

All the other dogs were negative and remained negative all the follow-up.
N/A*: Mortality related to CanL
N/A: Lost to follow-up

**Serological status**

Initially, all dogs were positive by both Witness® test and IFAT and no significant differences ($P > 0.05$) were observed between groups. The mean of *Leishmania*-antibody titers decreased over time (mean $\pm$ SD, 444.05 $\pm$ 467.50

At D0, 181.25 ± 234.71 at D+ 30, 120.69 ± 298.36 at D+ 90 and 118.97 ± 297.73 at D+ 180) after treatments (Figure 4). Significant reductions compared to baseline values ($P < 0.012$, IFAT serology) were observed in both groups at D+30 days (mean ± SD, artesunate 128.57 ± 212.78; Glucantime®/allopurinol 209.61 ± 244.96). They continued to decrease (mean ± SD, artesunate 37.50 ± 60.77; Glucantime®/ allopurinol 176.471 ± 379.604) at D+ 180 (Figure 3). When groups were compared, the artesunate group showed higher percentage of reduction in antibody titers at the end of the study ($P <0.0001$) (Figure 5). At the individual level, 78.57% (11/14) of dogs treated with Artesunate required diminution in antibody titers at D+ 30 and 50% (7/14) of them became absolutely seronegative. By contrast in the Glucantime®/ allopurinol group, 57.7% (15/26) required diminution in serology at D+ 30 and only 15.38% (4/26) became seronegative. At the end of the follow up, 41.37% (12/29) of dogs turned seronegative at the end of the follow-up including: 29.41% (5/17) in the Group 1 *versus* 58.33% (7/12) in the Group 2 (P-Value, Z test/bilateral= 0.227).

In conclusion, these results indicate that artesunate is an improved effective treatment against canine visceral leishmaniosis compared to the reference combination meglumine antimoniate/allopurinol. In addition, artesunate was safer easier to administrate (orally) with a short period-treatment, and is less expensive than meglumine antimoniate and allopurinol.

## Claims

1. A veterinary composition comprising artesunate or a salt thereof, and a pharmaceutically acceptable excipient, for use for preventing and/or treating leishmaniosis in a dog.

2. A veterinary composition for use according to claim 1, wherein said dog is a *Leishmania infantum* naturally infected dog.

3. A veterinary composition for use according to claim 1 or 2, wherein said veterinary comprises a therapeutically efficient dose comprised between 0.1 and 100 mg/kg BW/day, preferably between 1 and 50 mg/kg BW/day, more preferably between 20 and 30 mg/kg BW/day.

4. A veterinary composition for use according to any one of claims 1 to 3, wherein said veterinary composition comprises a therapeutically efficient dose of 25 mg/kg BW/day.

5. A veterinary composition for use according to any one of claims 1 to 4, wherein said veterinary composition is administered by an oral route.

6. A veterinary composition for use according to any one of claims 1 to 5, wherein said veterinary composition is a tablet.

7. A veterinary composition for use according to any one of claims 1 to 6, wherein said veterinary composition is administered in a single daily dose.

8. A veterinary composition for use according to any one of claims 1 to 7, wherein said veterinary composition is administered for at least 6 days, preferably from 6 to 10 days, more preferably for 6 days.

9. A veterinary composition for use according to any one of claims 1 to 8, for controlling the clinical signs of leishmaniosis.

10. A veterinary composition for use according to any one of claims 1 to 8, for controlling *Leishmania infantum* blood load.

11. A veterinary composition for use according to any one of claims 1 to 8, for controlling *Leishmania infantum* antibodies.

12. A veterinary composition comprising artesunate or a salt thereof, and a pharmaceutically acceptable excipient, for use for preventing and/or treating leishmaniosis in a *Leishmania infantum* naturally infected dog, wherein said veterinary composition is administered to said infected dog by oral route in a single daily dose of 25 mg/kg BW/day for 6 days.

13. A veterinary composition for use according to any one of claims 1 to 12, wherein said veterinary composition further comprises at least one antiparasitic agent.

14. A kit comprising a first compartment comprising artesunate or a salt thereof, and a second compartment comprising at least one antiparasitic agent as a combined preparation for simultaneous, separate, or sequential use, for preventing and/or treating leishmaniosis in a dog, said kit optionally comprises a notice or an instruction guide.

## Patentansprüche

1. Veterinärzusammensetzung, umfassend Artesunat oder ein Salz davon und einen pharmazeutisch verträglichen Hilfsstoff, zur Verwendung zum Vorbeugen und/oder Behandeln von Leishmaniose bei einem Hund.

2. Veterinärzusammensetzung zur Verwendung nach Anspruch 1, wobei der Hund ein mit *Leishmania infantum* natürlich infizierter Hund ist.

3. Veterinärzusammensetzung zur Verwendung nach Anspruch 1 oder 2, wobei die veterinäre eine therapeutisch wirksame Dosis umfasst, die zwischen 0,1 und 100 mg/kg KG/Tag, vorzugsweise zwischen 1 und 50 mg/kg KG/Tag, mehr bevorzugt zwischen 20 und 30 mg/kg KG/Tag umfasst ist.

4. Veterinärzusammensetzung zur Verwendung nach einem der Ansprüche 1 bis 3, wobei die Veterinärzusammensetzung eine therapeutisch wirksame Dosis von 25 mg/kg KG/Tag umfasst.

5. Veterinärzusammensetzung zur Verwendung nach einem der Ansprüche 1 bis 4, wobei die Veterinärzusammensetzung auf oralem Wege verabreicht wird.

6. Veterinärzusammensetzung zur Verwendung nach einem der Ansprüche 1 bis 5, wobei die Veterinärzusammensetzung eine Tablette ist.

7. Veterinärzusammensetzung zur Verwendung nach einem der Ansprüche 1 bis 6, wobei die Veterinärzusammensetzung in einer einzigen Tagesdosis verabreicht wird.

8. Veterinärzusammensetzung zur Verwendung nach einem der Ansprüche 1 bis 7, wobei die Veterinärzusammensetzung für mindestens 6 Tage, vorzugsweise 6 bis 10 Tage, mehr bevorzugt für 6 Tage, verabreicht wird.

9. Veterinärzusammensetzung zur Verwendung nach einem der Ansprüche 1 bis 8 zum Bekämpfen der klinischen Anzeichen von Leishmaniose.

10. Veterinärzusammensetzung zur Verwendung nach einem der Ansprüche 1 bis 8 zum Bekämpfen einer Blutbelastung durch *Leishmania infantum.*

11. Veterinärzusammensetzung zur Verwendung nach einem der Ansprüche 1 bis 8 zum Bekämpfen von Antikörpern von*Leishmania-infantum.*

12. Veterinärzusammensetzung, umfassend Artesunat oder ein Salz davon und einen pharmazeutisch verträglichen Hilfsstoff, zur Verwendung zum Vorbeugen und/oder Behandeln von Leishmaniose bei einem mit *Leishmania infantum* natürlich infizierten Hund, wobei die Veterinärzusammensetzung dem infizierten Hund auf oralem Wege in einer einzigen Tagesdosis von 25 mg/kg KG/Tag für 6 Tage verabreicht wird.

13. Veterinärzusammensetzung zur Verwendung nach einem der Ansprüche 1 bis 12, wobei die Veterinärzusammensetzung ferner mindestens ein antiparasitäres Mittel umfasst.

14. Kit, umfassend ein erstes Kompartiment, umfassend Artesunat oder ein Salz davon, und ein zweites Kompartiment, umfassend mindestens ein antiparasitäres Mittel, als ein kombiniertes Präparat zur gleichzeitigen, getrennten oder aufeinanderfolgenden Verwendung zum Vorbeugen und/oder Behandeln von Leishmaniose bei einem Hund, wobei das Kit optional einen Hinweis oder eine Gebrauchsanweisung umfasst.

## Revendications

1. Composition vétérinaire comprenant de l'artésunate ou un sel de celui-ci, et un excipient pharmaceutiquement acceptable, pour une utilisation dans la prévention et/ou le traitement de la leishmaniose chez un chien.

2. Composition vétérinaire pour une utilisation selon la revendication 1, dans laquelle ledit chien est un chien naturellement infecté par *Leishmania infantum.*

3. Composition vétérinaire pour une utilisation selon la revendication 1 ou 2, dans laquelle ledit vétérinaire comprend une dose thérapeutiquement efficace comprise entre 0,1 et 100 mg/kg de poids corporel par jour, de préférence entre 1 et 50 mg/kg de poids corporel par jour, plus préférablement entre 20 et 30 mg/kg de poids corporel par jour.

4. Composition vétérinaire pour une utilisation selon l'une quelconque des revendications 1 à 3, dans laquelle ladite composition vétérinaire comprend une dose thérapeutiquement efficace de 25 mg/kg de poids corporel par jour.

5. Composition vétérinaire pour une utilisation selon l'une quelconque des revendications 1 à 4, dans laquelle ladite composition vétérinaire est administrée par voie orale.

6. Composition vétérinaire pour une utilisation selon l'une quelconque des revendications 1 à 5, dans laquelle ladite composition vétérinaire est un comprimé.

7. Composition vétérinaire pour une utilisation selon l'une quelconque des revendications 1 à 6, dans laquelle ladite composition vétérinaire est administrée en une seule dose quotidienne.

8. Composition vétérinaire pour une utilisation selon l'une quelconque des revendications 1 à 7, dans laquelle ladite composition vétérinaire est administrée pendant au moins 6 jours, de préférence de 6 à 10 jours, plus préférablement pendant 6 jours.

9. Composition vétérinaire pour une utilisation selon l'une quelconque des revendications 1 à 8, pour contrôler les signes cliniques de la leishmaniose.

10. Composition vétérinaire pour une utilisation selon l'une quelconque des revendications 1 à 8, pour contrôler la charge sanguine en *Leishmania infantum.*

11. Composition vétérinaire pour une utilisation selon l'une quelconque des revendications 1 à 8, pour contrôler les anticorps de *Leishmania infantum.*

12. Composition vétérinaire comprenant de l'artésunate ou un sel de celui-ci, et un excipient pharmaceutiquement acceptable, pour une utilisation dans la prévention et/ou le traitement de la leishmaniose chez un chien naturellement infecté par *Leishmania infantum,* dans laquelle ladite composition vétérinaire est administrée audit chien infecté par voie orale en une seule dose quotidienne de 25 mg/kg de poids corporel par jour pendant 6 jours.

13. Composition vétérinaire pour une utilisation selon l'une quelconque des revendications 1 à 12, dans laquelle ladite composition vétérinaire comprend en outre au moins un agent antiparasitaire.

14. Kit comprenant un premier compartiment comprenant de l'artésunate ou un sel de celui-ci, et un second compartiment comprenant au moins un agent antiparasitaire sous forme de préparation combinée pour une utilisation simultanée, séparée ou séquentielle, afin de prévenir et/ou de traiter la leishmaniose chez un chien, ledit kit comprenant éventuellement une notice ou un guide d'instruction.

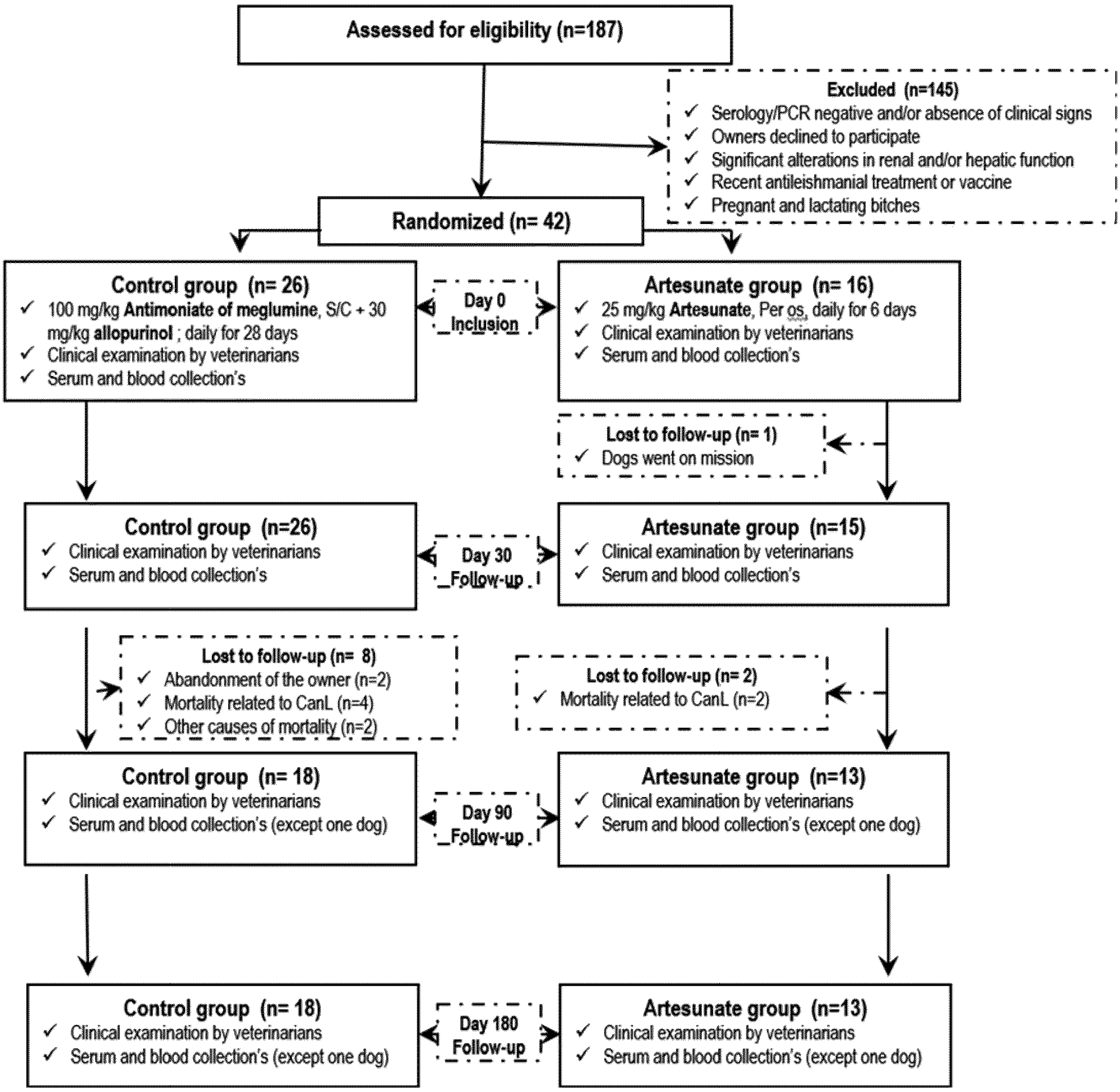
Assessed for eligibility (n=187)
Excluded (n=145)
Serology/PCR negative and/or absence of clinical signs
Owners declined to participate
Significant alterations in renal and/or hepatic function
Recent antileishmanial treatment or vaccine
Pregnant and lactating bitches
Randomized (n= 42)
Control group (n= 26)
100 mg/kg Antimoniate of meglumine, S/C + 30 mg/kg allopurinol ; daily for 28 days
Clinical examination by veterinarians
Serum and blood collection's
Day 0
Inclusion
Artesunate group (n= 16)
25 mg/kg Artesunate, Per os, daily for 6 days
Clinical examination by veterinarians
Serum and blood collection's
Lost to follow-up (n= 1)
Dogs went on mission
Control group (n=26)
Clinical examination by veterinarians
Serum and blood collection's
Day 30
Follow-up
Artesunate group (n=15)
Clinical examination by veterinarians
Serum and blood collection's
Lost to follow-up (n= 8)
Abandonment of the owner (n=2)
Mortality related to CanL (n=4)
Other causes of mortality (n=2)
Lost to follow-up (n= 2)
Mortality related to CanL (n=2)
Control group (n= 18)
Clinical examination by veterinarians
Serum and blood collection's (except one dog)
Day 90
Follow-up
Artesunate group (n=13)
Clinical examination by veterinarians
Serum and blood collection's (except one dog)
Control group (n= 18)
Clinical examination by veterinarians
Serum and blood collection's (except one dog)
Day 180
Follow-up
Artesunate group (n=13)
Clinical examination by veterinarians
Serum and blood collection's (except one dog)

Figure 1

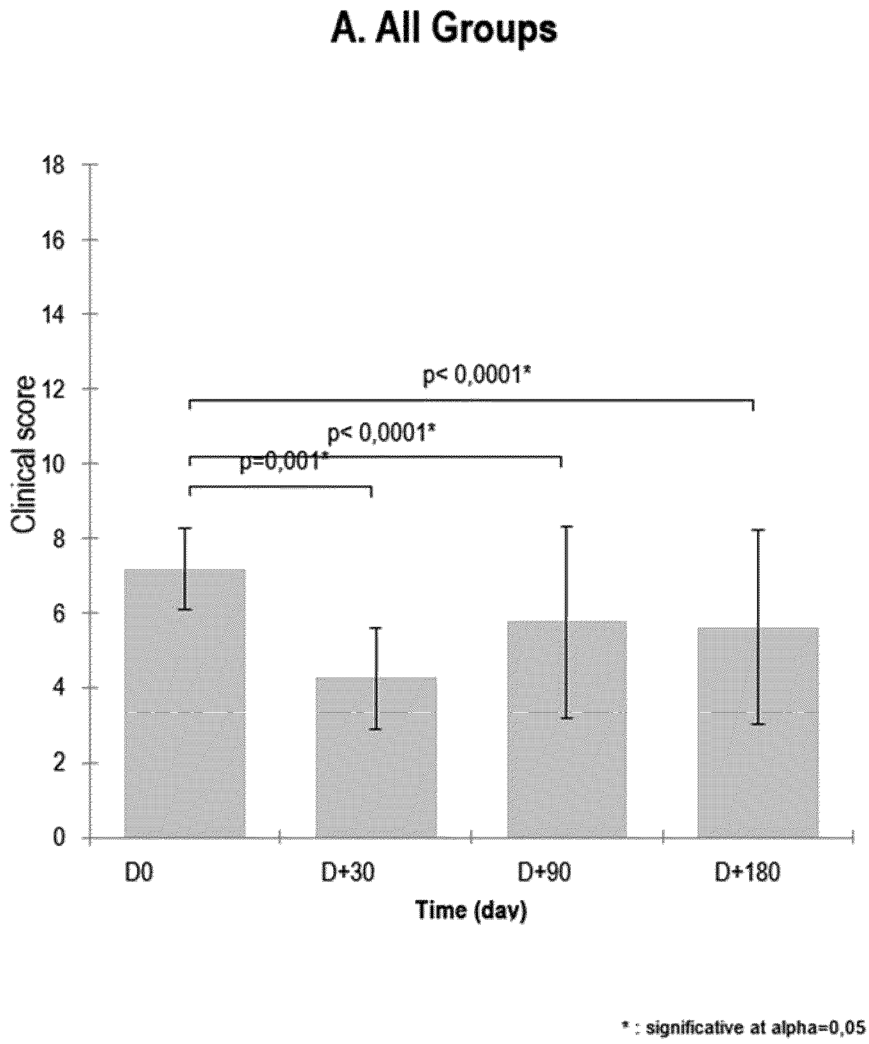
A. All Groups
18
16
14
12
10
8
6
4
2
0
Clinical score
p< 0,0001*
p< 0,0001*
p=0,001*
D0
D+30
D+90
D+180
Time (day)
* : significative at alpha=0,05

Figure 2

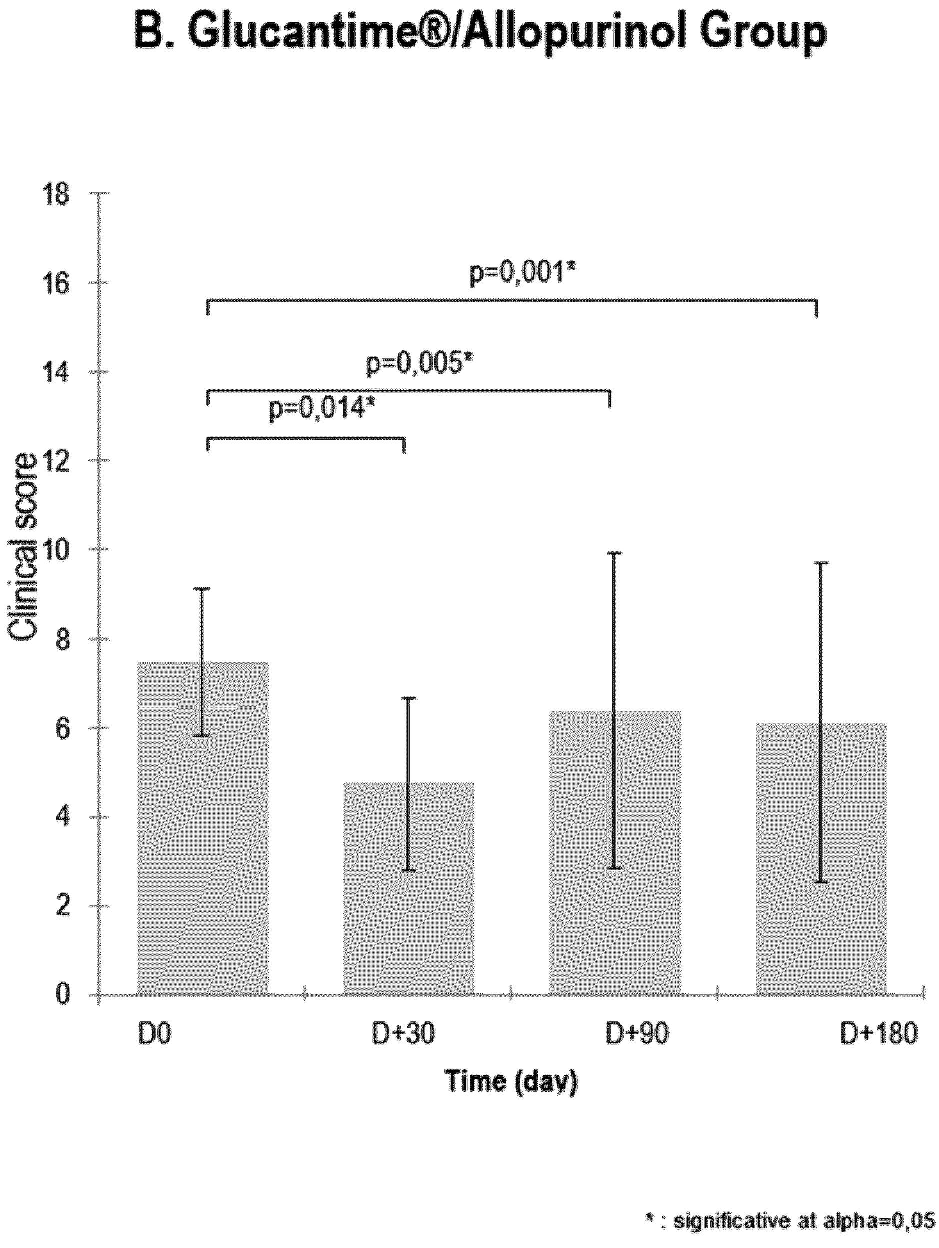


**Figure 2 Cont.**

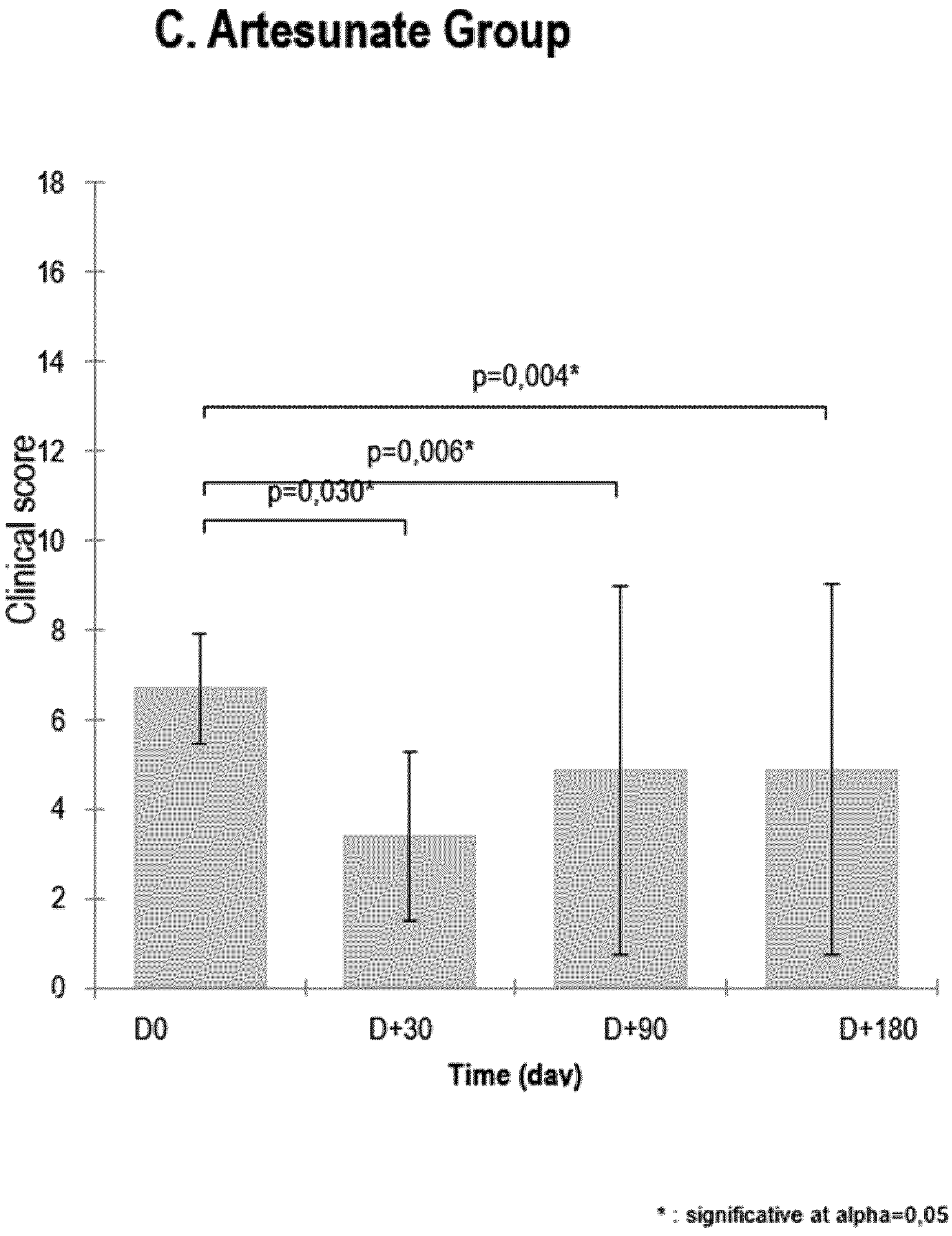


Figure 2 Cont.

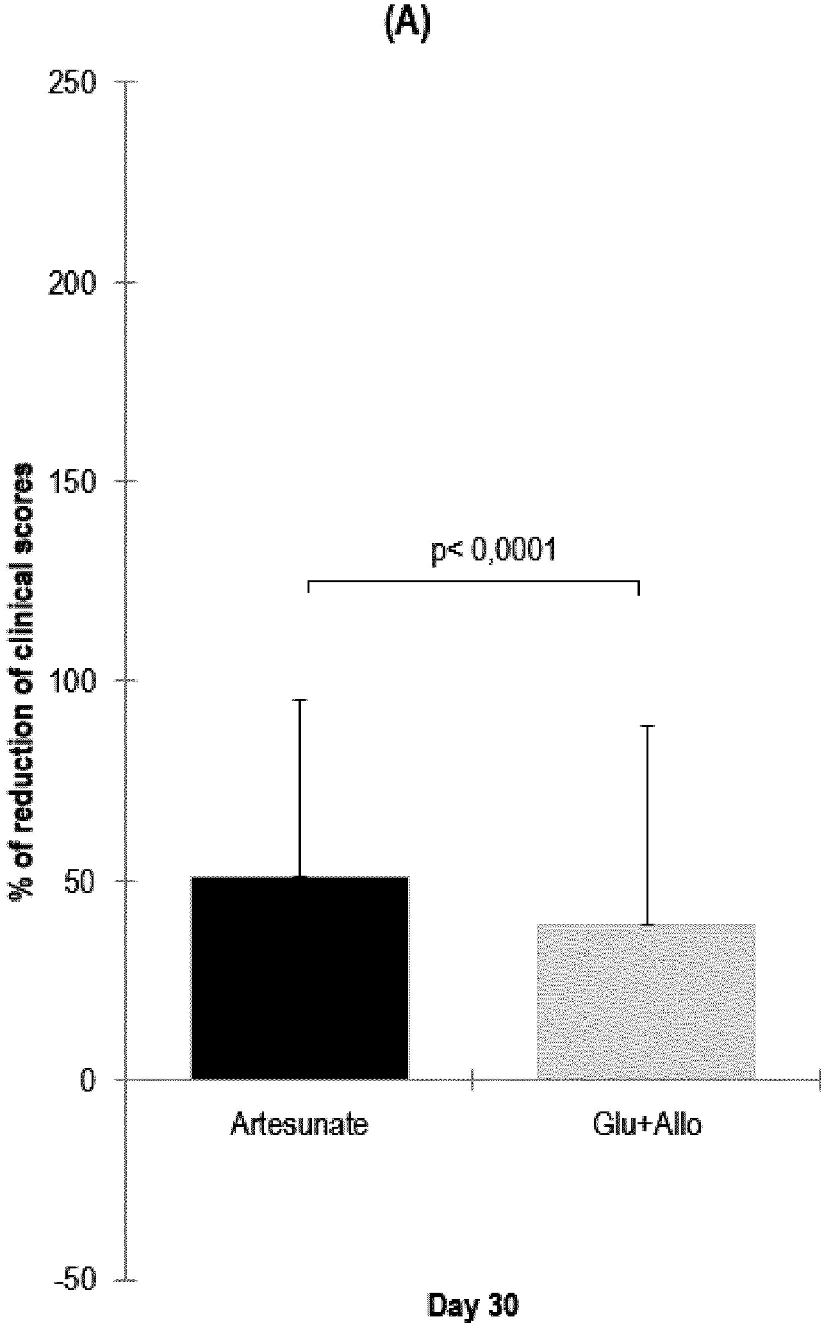
(A)
250
200
150
100
50
0
-50
% of reduction of clinical scores
p< 0,0001
Artesunate
Glu+Allo
Day 30

Figure 3

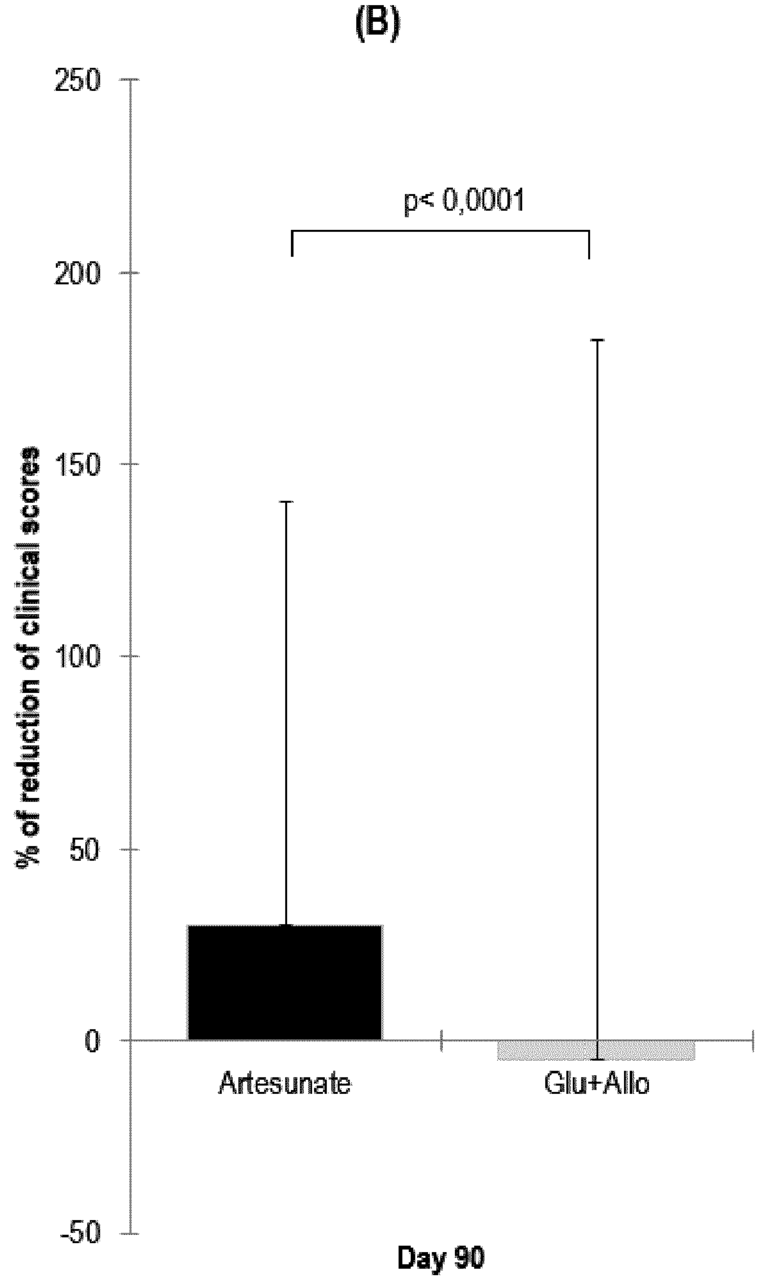


Figure 3 Cont.

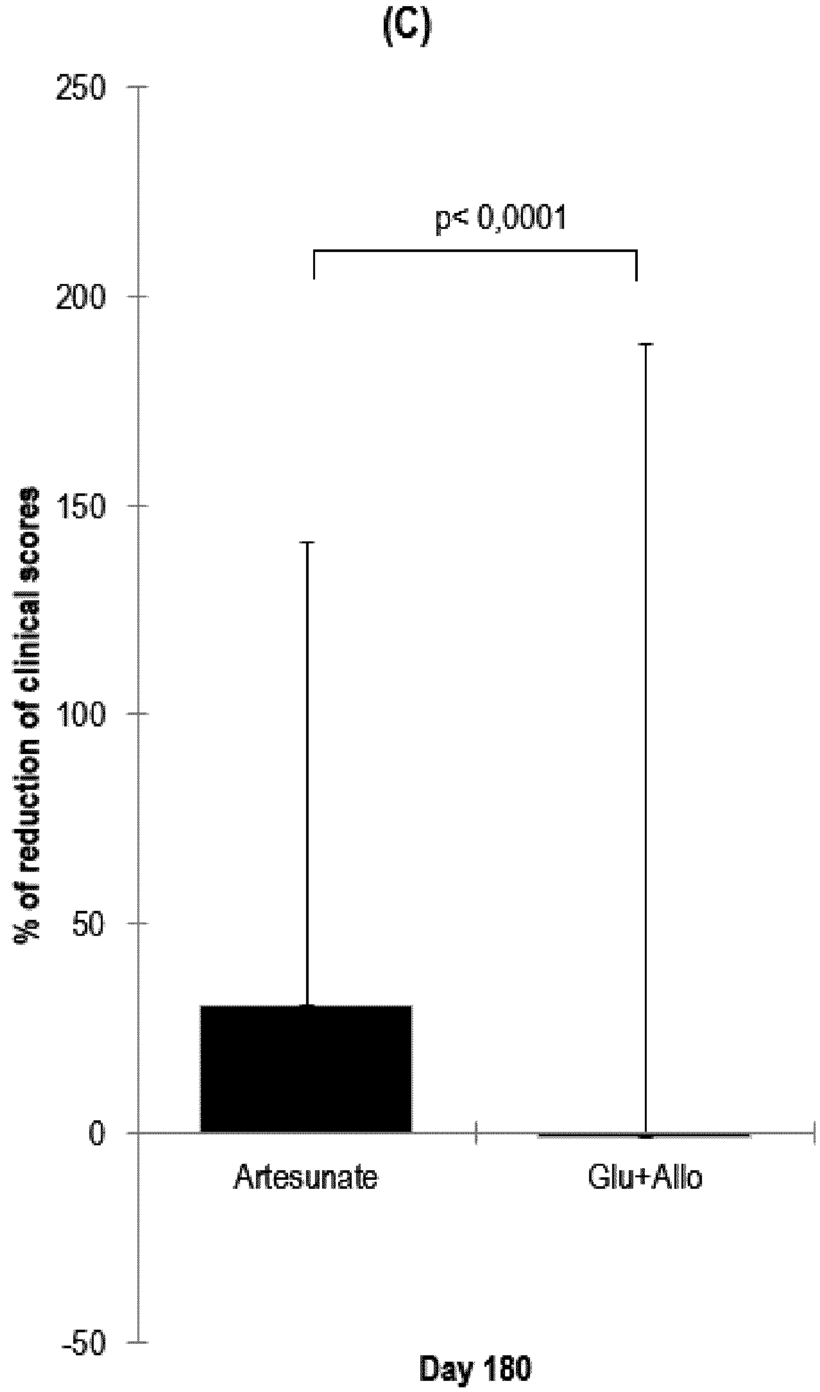


Figure 3 Cont.

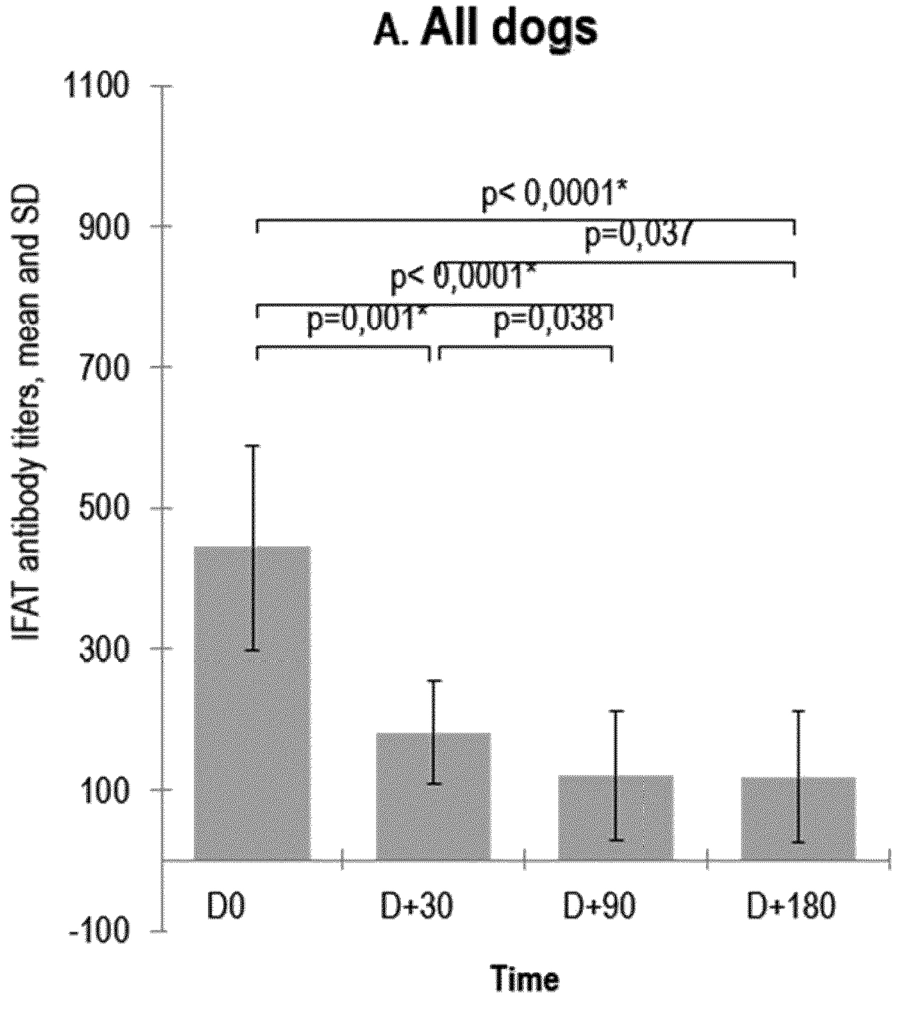


* : significative at alpha=0,05

Figure 4

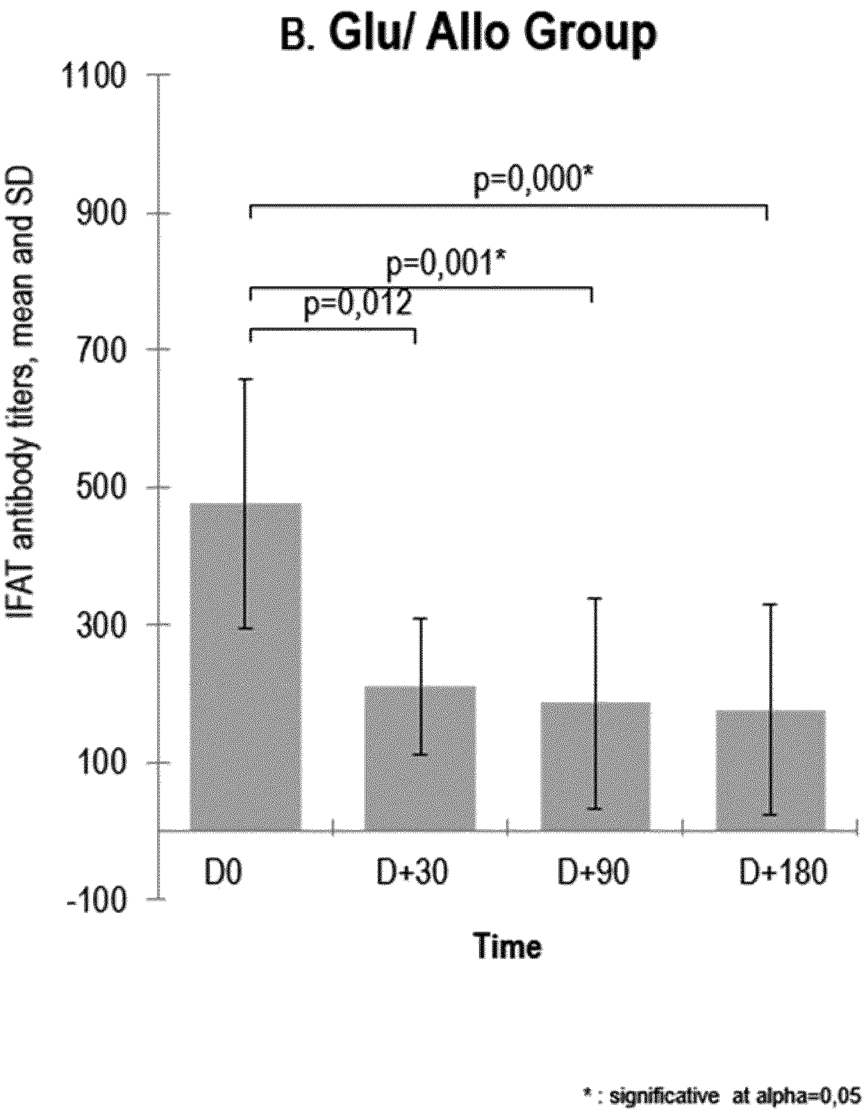


**Figure 4 Cont.**

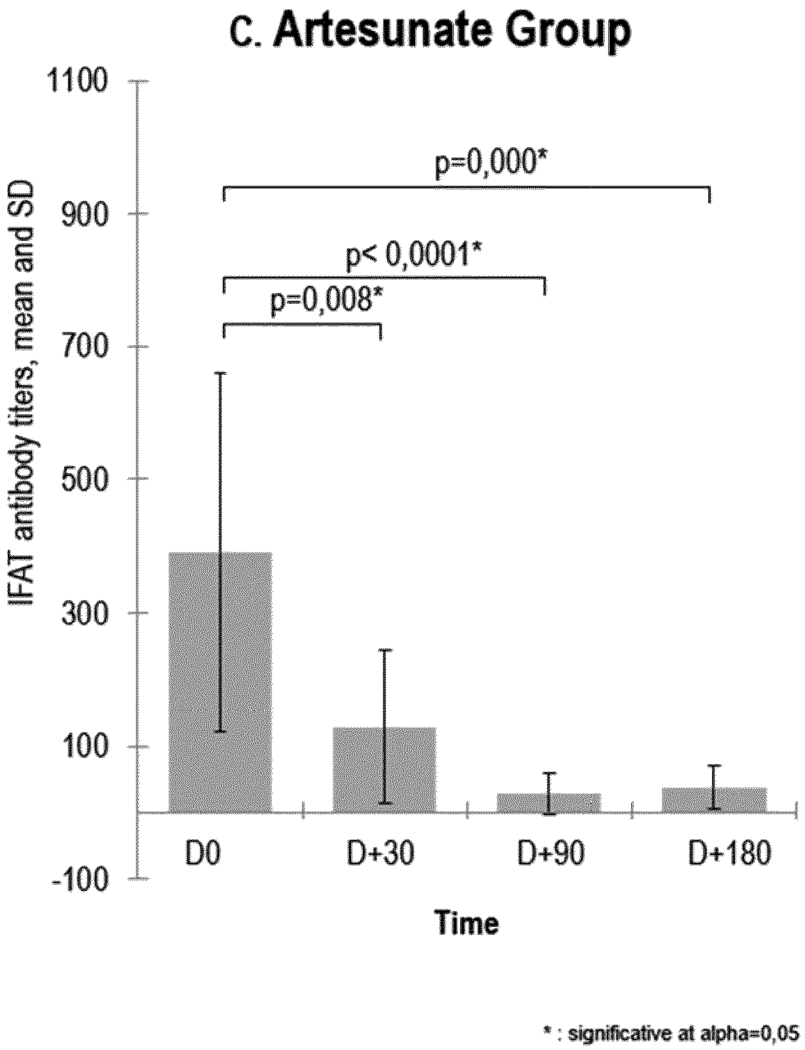


Figure 4 Cont.

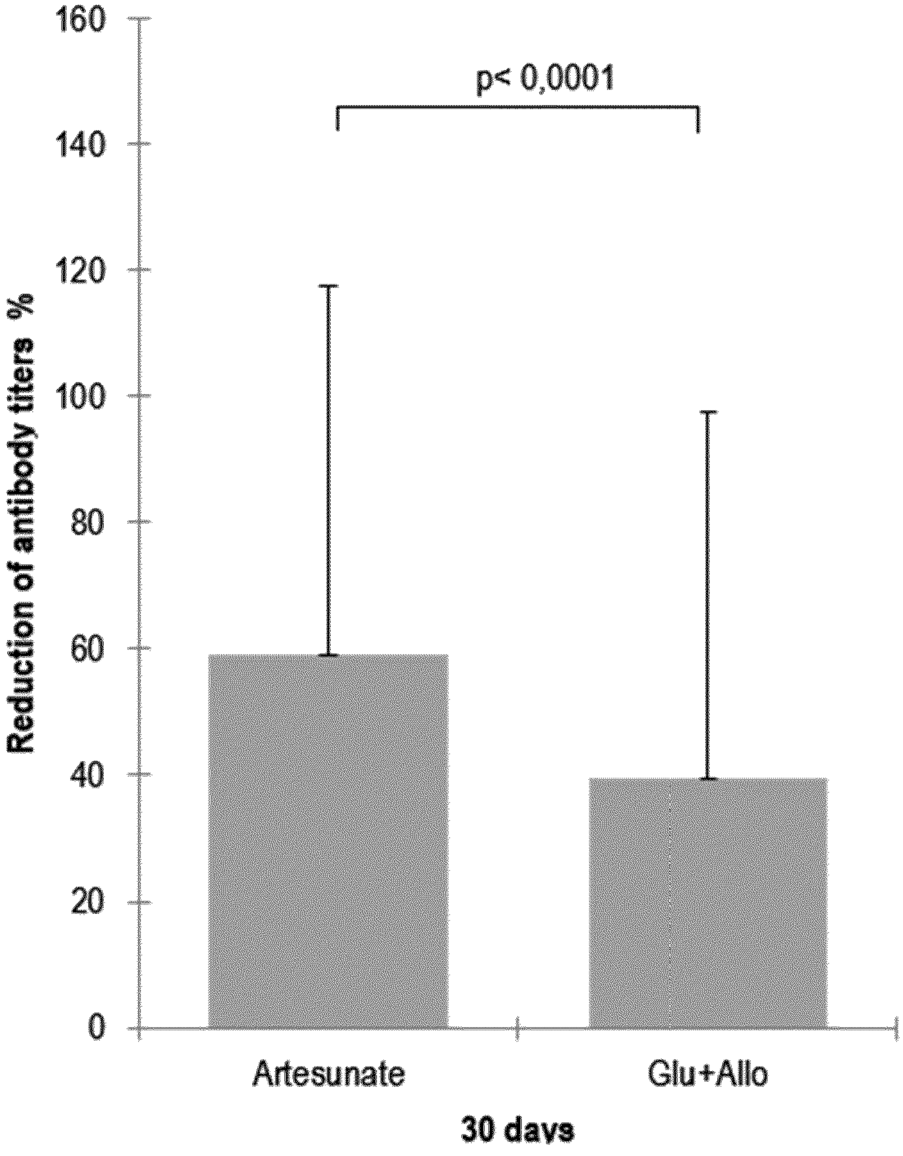
160
140
120
100
80
60
40
20
0
Reduction of antibody titers %
p< 0,0001
Artesunate
Glu+Allo
30 days

Figure 5

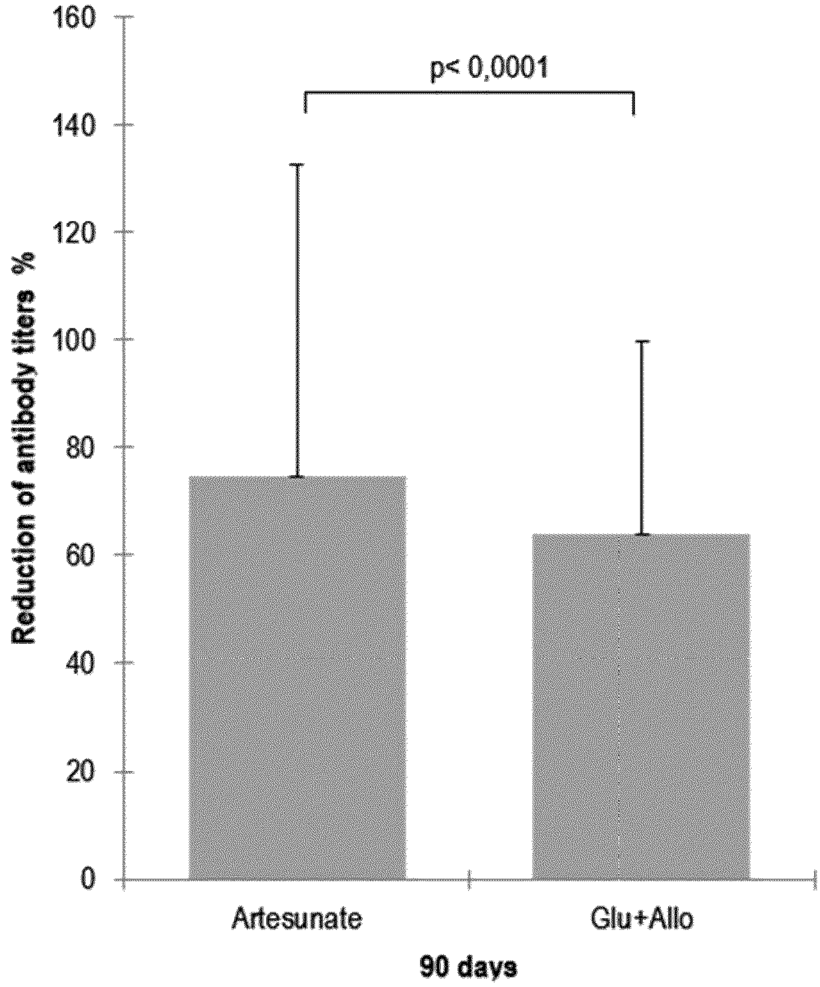


Figure 5 Cont.

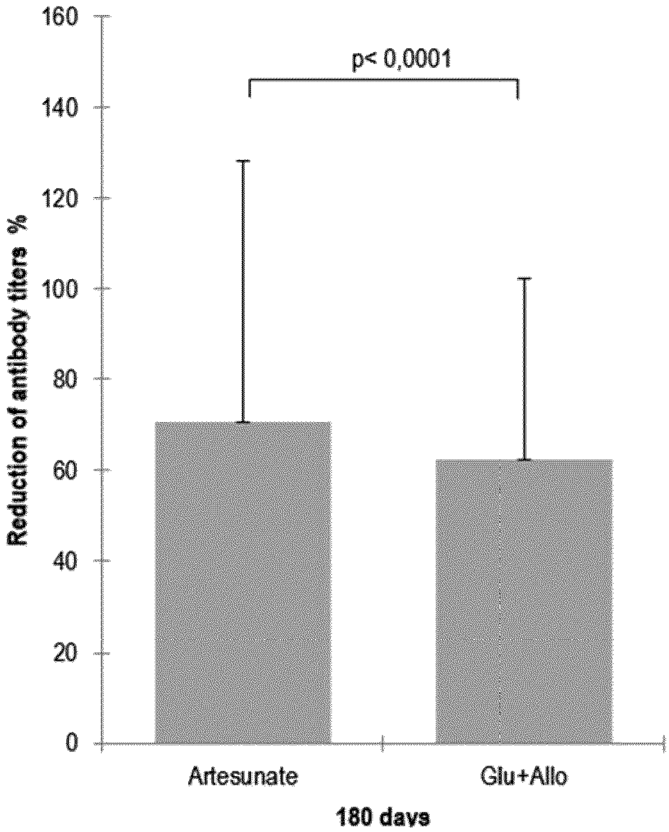


Figure 5 Cont.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- WHO Model List of Essential Medicines. April 2015
- **BOURDOISEAU ; DENEROLLE.** *Revue Méd. Vét.,* 2000, vol. 151 (5), 395-400
- **SOLANO-GALLEGO et al.** *Parasites & Vectors,* 2011, vol. 4, 86
- **LOO et al.** *Pharmacol Res.,* 2017, vol. 117, 192-217
- **CHOLLET et al.** *Biomedicine & Pharmacotherapy,* 2008, vol. 62, 462-465
- **CORTES et al.** *Antimicrobial agents and chemotherapy,* 2015, vol. 59 (8), 5032-5035